(19) Europäisches Patentamt
European Patent Office
Office européen des brevets

(11) Veröffentlichungsnummer: **0 481 253 B1**

(12) **EUROPÄISCHE PATENTSCHRIFT**

(45) Veröffentlichungstag der Patentschrift: **28.12.94**

(51) Int. Cl.$^5$: **C07C 65/24**, A61K 31/19, C07C 69/92, C07D 319/00

(21) Anmeldenummer: **91116325.1**

(22) Anmeldetag: **25.09.91**

(54) **Neue p-Oxibenzoesäurederivate, Verfahren zu ihrer Herstellung und ihre Verwendung als Arzneimittel.**

(30) Priorität: **09.10.90 DE 4032037**

(43) Veröffentlichungstag der Anmeldung:
**22.04.92 Patentblatt 92/17**

(45) Bekanntmachung des Hinweises auf die Patenterteilung:
**28.12.94 Patentblatt 94/52**

(84) Benannte Vertragsstaaten:
**AT BE CH DE DK ES FR GB GR IT LI LU NL SE**

(56) Entgegenhaltungen:
**EP-A- 0 133 935**
**EP-A- 0 286 703**
**DE-A- 2 460 689**

(73) Patentinhaber: **Klinge Pharma GmbH**
**Berg-am-Laim-Strasse 129**
**D-81673 München (DE)**

(72) Erfinder: **Reiter, Friedemann, Dr.**
**Zugspitzstrasse 36**
**W-8011 Putzbrunn (DE)**
Erfinder: **Jank, Peter, Dr.**
**Kiefernstrasse 13**
**W-8011 Hofolding (DE)**
Erfinder: **Schliack, Michael**
**Neumarkter Strasse 82**
**W-8000 München 80 (DE)**
Erfinder: **Kaipainen-Reiter, Kaisli**
**Zugspitzstrasse 36**
**W-8011 Putzbrunn (DE)**
Erfinder: **Lang, Gerhard**
**Landshuter Strasse 114**
**W-8050 Freising (DE)**
Erfinder: **Schink, Anke**
**Winterstrasse 3**
**W-8011 Grasbrunn (DE)**

(74) Vertreter: **von Hellfeld, Axel, Dr. Dipl.-Phys. et al**
**Wuesthoff & Wuesthoff**
**Patent- und Rechtsanwälte**
**Schweigerstrasse 2**
**D-81541 München (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.10/3.09/3.3.3)

## Beschreibung

Aus der DE-OS 33 26 164 und dem Europäischen Patent 0 133 935 sind therapeutisch wertvolle p-Oxibenzoesäurederivate der allgemeinen Formel

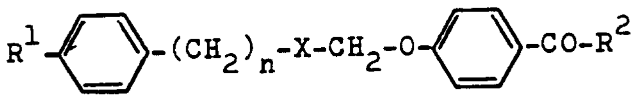

in der bedeuten:

$R^1$ =

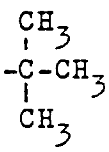 oder 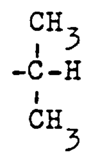

n = 1 oder 2

X =

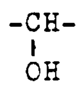 oder 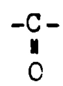

und

$R^2$ = -OH oder $-NHCH_2COOH$

sowie ihre physiologisch verträglichen Salze bekannt.

Aus der DE-OS 33 26 164 und dem Europäischen Patent 0 133 935 sind ferner Verfahren zur Herstellung der vorgenannten Verbindungen und ihre Verwendung zur Herstellung eines Arzneimittels mit hypolipämischer Wirkung bekannt.

Bekannt ist auch, daß bestimmte, in Paraposition veretherte Benzoesäuren hypolipämische Eigenschaften besitzen, nämlich Benzoesäurederivate die sich von Ethern des Glycerins [ DE-PS 24 60 689] oder des 1,3-Dihydroxiacetons [ DE-OS 27 35 856 ] ableiten.

Überraschenderweise wurde nun gefunden, daß die vorteilhaften pharmakodynamischen und pharmakokinetischen Eigenschaften noch ausgeprägter in Erscheinung treten können, wenn in Abwandlung der bekannten Verbindungen der DE-OS 33 26 164 andere Substituenten für $R^1$ bzw. $R^2$ verwendet werden.

Gegenstand der vorliegenden Erfindung sind p-Oxibenzoesäurederivate der allgemeinen Formel (1)

(1) 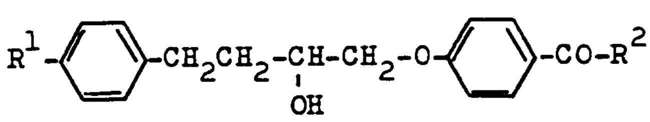

ihre physiologisch verträglichen Salze, deren Enantiomere oder Diastereomere, wobei

$R^1$ =

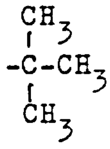 , 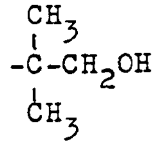 oder 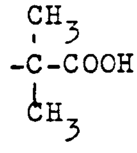

und $R^2$ = -OH,

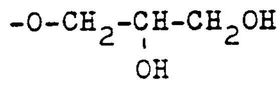 oder 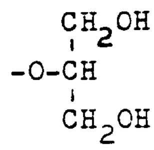

wobei im Falle von $R^1$ =

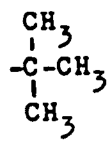

$R^2$ =

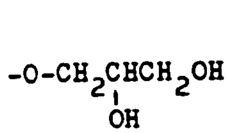 oder 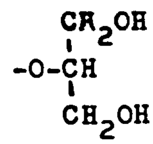

bedeutet
und im Falle von $R^1$ =

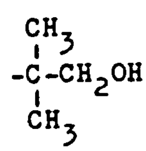 oder 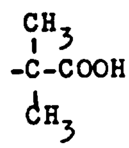

$R^2$ = -OH
bedeuten können.

Gegenstand der Erfindung ist ferner eine Verfahren zur Herstellung der erfindungsgemäßen Verbindungen, das dadurch gekennzeichnet ist, daß man Epoxide der allgemeinen Formel (2)

(2) 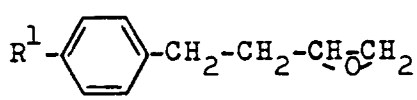

in der $R^1$ =

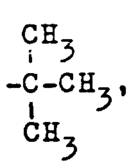 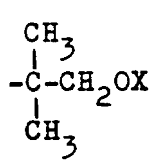 oder 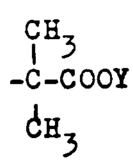

bedeutet und sowohl X als auch Y eine geeignete Schutzgruppe, z.B. Acetyl, darstellt mit den entsprechenden p-Hydroxibenzoesäureestern in Gegenwart von Alkali umsetzt und die gegebenenfalls vorhandenen Schutzgruppen abspaltet. Dabei können Methoden angewandt werden, wie sie aus der DE-OS 33 26 164 bekannt sind.

Die folgenden Beispiele sollen die Erfindung näher erläutern.

Es wurden die in der folgenden Tabelle 1 aufgeführten Verbindungen festgestellt:

**Tabelle 1**

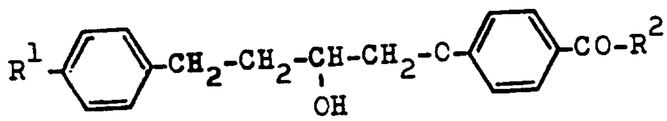

| Nr. | $R^1$ | $R^2$ |
|---|---|---|
| 1 | $(CH_3)_3C$ | $OCH_2CHCH_2OH$<br>OH |
| 2 | $HOCH_2C(CH_3)_2$ | OH |
| 3 | $HOOCC(CH_3)_2$ | OH |

**Beispiel 1**

**4-[4-(4'-tert.Butylphenyl)-2-hydroxybutoxy]benzoesäure-2,3-dihydroxypropylester**

**a) 4-[4-(4'-tert.Butylphenyl)-2-hydroxybutoxy]benzoesäure-(2,2-dimethyl-1,3-dioxolan-4-yl)-methylester**

Zur Lösung von 27.7 g (0.110 Mol) 4-Hydroxybenzoesäure-(2,2-dimethyl-1,3-dioxolan-4-yl)methylester in 200 ml absolutem Dimethyformamid fügt man bei Raumtemperatur unter Rühren 0.300g (0.010 Mol) 80%iges Natriumhydrid und nach Beendigung der Gasentwicklung 20.4g (0.100 Mol) 4-(4'-tert.Butylphenyl)-1,2-epoxybutan. Die Mischung wird 20 Stunden auf 100-110 °C erhitzt. Nach Abkühlen der Reaktionsmischung gießt man in Eiswasser, säuert die Lösung an, nimmt das Produkt in Ethylacetat auf und wäscht die organische Phase mit Wasser. Nach dem Trocknen über Natriumsulfat wird das Lösungsmittel i. Vak. entfernt Man erhält 47.2g gelbbraunes, zähflüssiges Rohprodukt, das ohne Reinigung zur weiteren Umsetzung verwendet wird.
$^1$H-NMR-Spektrum ($CDCl_3$):
1.31 s (9) $(CH_3)_3C$
1.39, 1.45 2s (6) $(CH_3)_2C$
1.73 bis 2.05 m (2) $ArCH_2CH_2$
2.35 d (1) OH
2.57 bis 3.00 m (2) $ArCH_2CH_2$
3.70 bis 4.23 m (5) $CH_2CHO$, $CH_2O$
4.27 bis 4.53 m (3) $COOCH_2CH$
6.80 bis 8.15 m (8) Aromat
NMR-Spektrum aufgenommen bei 100 MHz
Die chemischen Verschiebungen sind in ppm gegen TMS ($\delta$ = 0.0) angegeben, die relativen Intensitäten

sind in Klammern beigefügt s = Singulett; d = Dublett; t = Triplett; q = Quartett; m = Multiplett.

**b) Herstellung von 4-[4-(4'-tert.Butylphenyl)-2-hydroxybutoxy]benzoesäure-2,3-dihydroxypropylester**

Die Lösung von 47.2g rohem 4-[4-(4'-tert.Butylphenyl)-2-hydroxybutoxy]benzoesäure-(2,2-dimethyl-1,3-dioxolan-4-yl)methylester in 200 ml Tetrahydrofuran wird mit 35 ml 5M Salzsäure versetzt und 2 Stunden bei Raumtemperatur gerührt. Man gießt in Eiswasser, nimmt das Produkt in Ethylacetat auf und wäscht mit Wasser. Nach dem Trocknen über Natriumsulfat wird das Losungsmittel i. Vak. entfernt. Der ölige, allmählich kristallisierende Rückstand wird viermal aus Ethylacetat umkristallisiert Farblose Kristalle vom Schmp. 101-103 ° C; Ausbeute 18.1 g (43%).
$C_{21}H_{32}O_6$ (416.5)
Molmasse 416 (massenspektrometrisch mittels Elektronenstoß-Ionisation (70 eV) bestimmt)
IR-Spektrum (KBr): $\nu$ (OH) 3640-3120 $cm^{-1}$
$\nu$ (C=O) 1710 $cm^{-1}$
$^1$H-NMR-Spektrum ($CDCl_3$):
1.30 s (9) $(C\underline{H}_3)_3C$
1.75 bis 2.04 m (2) $ArCH_2C\underline{H}_2$
2.63 bis 3.10 m (4) $ArC\underline{H}_2CH_2$, 2 O$\underline{H}$
3.50 d (1) O$\underline{H}$
3.57 bis 4.20 m (6) 2 $C\underline{H}_2C\underline{H}O$
4,35 d (2) $COOC\underline{H}_2$
6.70 bis 8.03 m (8) Aromat

## Beispiel 2

### 4-{4-[4'-(2-Hydroxy-1,1-dimethylethyl)phenyl]-2-hydroxybutoxy}benzoesäure

**a) 4-[4-{4'-[1,1-Dimethyl-2-(tetrahydropyran-2-yl)oxyethyl]phenyl}-2-hydroxybutoxybenzoesäure-methylester**

30.4g (0.100 Mol) 4-{4'-[1,1-Dimethyl-2-(tetrahydropyran-2-yl)oxyethyl]phenyl}-1,2-epoxybutan, 15.2 g (0.100 Mol) 4-Hydroxybenzoesäure-methylester und 1.74g (0.010 Mol) 4-Hydroxybenzoesäure-methylester Natriumsalz werden in 100 ml Dimethylformamid 23 Stunden unter Rühren auf 100-110 ° C erhitzt. Die abgekühlte Reaktionsmischung wird in Wasser gegossen, das sich abscheidende Produkt in Ethylacetat aufgenommen und die organische Phase mit Wasser gewaschen. Nach dem Trocknen über Natriumsulfat wird das Lösungsmittel i. Vak. entfernt und der Rückstand mit Dichlormethan/Methanol 99/1 an Kieselgel chromatographiert. Man erhält ein hochviskoses Öl Ausbeute 32.5g (71%).
$^1$H-NMR-Spektrum ($CDCl_3$), (60 MHz):
1.13 bis 2.27 m (14) $(C\underline{H}_3)_2C$, $(C\underline{H}_2)_3$, $ArCH_2C\underline{H}_2$
2.50 bis 3.03 m (3) $ArC\underline{H}_2CH_2$, O$\underline{H}$
3.77 bis 4.33 m (10) $C\underline{H}_2C\underline{H}O$, 2 $C\underline{H}_2O$, $C\underline{H}_3O$
4.50 breites s (1) $OC\underline{H}O$
6.67 bis 8.10 m (8) Aromat

**b) 4-[4-{4'-[1,1-Dimethyl-2-(tetrahydropyran-2-yl)oxyethyl]phenyl}- 2-hydroxybutoxybenzoesäure**

32.5 g (0.071 Mol) 4-[4-{4'-[1,1-Dimethyl-2-(tetrahydropyran-2-yl)oxyethyl]phenyl}-2-hydroxybutoxybenzoesäure-methylester in 130 ml Methanol werden mit einer Losung von 16.0 g (0.230 Mol) Kaliumhydroxid in 55 ml Wasser versetzt und 3 Stunden unter Rückfluß erhitzt. Anschließend wird das Lösungsmittel i. Vak. entfernt, der Rückstand in Wasser aufgenommen, mit Ether extrahiert und die wässrige Phase mit konz. Salzsäure angesäuert. Das sich abscheidende Produkt wird in Ethylacetat aufgenommen und die organische Phase mit Wasser gewaschen. Nach dem Trocknen über Natriumsulfat wird das Lösungsmittel i. Vak. entfernt. Man erhält 31.2 g gelbes Harz als Rückstand (Rohausbeute 99%).

**c) Herstellung von 4-{4-[4'-(2-Hydroxy-1,1-dimethylethyl)phenyl]-2-hydroxybutoxy}benzoesäure**

31.2g (0.070 Mol) 4-[4-{4'-[1,1-Dimethyl-2-(tetrahydropyran-2-yl)oxyethyl]phenyl }-2-hydroxybutoxybenzoesäure werden in 100 ml Methanol gelöst und mit 10 ml konz. Salzsäure 1 Stunde bei 40-50 °C gerührt. Anschließend wird mit Wasser verdünnt, das sich abscheidende Produkt wird in Ethylacetat aufgenommen und die organische Phase mit Wasser gewaschen. Nach dem Trocknen über Natriumsulfat wird das Lösungsmittel i. Vak. entfernt und der Rückstand dreimal aus Acetonitril/Trichlormethan (9/1) umkristallisiert Farblose Kristalle vom Schmp. 118-119 °C; Ausbeute 13.0g (52%).
$C_{21}H_{26}O_5$ (358.4)
Massenspektrum: m/e = 327(100%) ($M^+$-$CH_2OH$) (kein $M^+$)
IR-Spektrum(KBr): $\nu$ (OH) 3600-2400 $cm^{-1}$
$\nu$ (C=O) 1675 $cm^{-1}$
$^1$H-NMR-Spektrum ($CDCl_3$):
1.31 s (6) $(C\underline{H}_3)_2C$
1.75 bis 2.10 m (2) $ArCH_2C\underline{H}_2$
2.58 bis 2.97 m (2) $ArC\underline{H}_2CH_2$
3.58 s (2) $C\underline{H}_2OH$
3.82 bis 4.30 m (6) $C\underline{H}_2C\underline{H}O$, 3 O$\underline{H}$
6.77 bis 8.10 m (8) Aromat

**<u>Beispiel 3</u>**

**<u>4-{4-[4'-(1-Carboxy-1-methylethyl)phenyl]-2-hydroxybutoxy}benzoesäure</u>**

**a) 4-{4-[4'-(1-Methoxycarbonyl-1-methylethyl)phenyl]-2-hydroxybutoxy}benzoesäure-methylester**

24.8 g (0.100 Mol) 4-[4'-(1-Methoxycarbonyl-1-methylethyl)phenyl]-1,2-epoxybutan, 15.2 g (0.100 Mol)4-Hydroxybenzoesäure-methylester und 1.74 g (0.010 Mol) 4-Hydroxybenzoesäure-methylester Natriumsalz werden in 100 ml Dimethylformamid 12 Stunden bei 100-110 °C gerührt. Die abgekühlte Reaktionsmischung wird in Wasser gegossen, das sich abscheidende Produkt wird in Ethylacetat aufgenommen und die organische Phase mit Wasser gewaschen. Nach dem Trocknen über Natriumsulfat wird das Lösungsmittel i. Vak. entfernt und der Rückstand (39.1 g, Rohausbeute 98%) ohne Reinigung weiter umgesetzt.
$^1$H-NMR-Spektrum ($CDCl_3$) (60 MHz):
1.57 s (6) $(C\underline{H}_3)_2C$
1.73 bis 2.20 m (2) $ArCH_2C\underline{H}_2$
2.50 bis 3.20 m (3) $ArC\underline{H}_2C\underline{H}_2$, O$\underline{H}$
3.63 s (3) $OC\underline{H}_3$
3.70 bis 4.20 m (6) $C\underline{H}_2C\underline{H}O$, $C\underline{H}_3O$
6.63 bis 8.10 m (8) Aromat

**b) Herstellung von 4-{4-[4'-(1-Carboxy-1-methylethyl)phenyl]-2-hydroxybutoxy}benzoesäure**

39.1 g (0.098 Mol) roher 4-{4-[4'-(1-Methoxycarbonyl-1-methylethyl)phenyl]-2-hydroxybutoxy}benzoesäure-methylester in 150 ml Methanol werden mit einer Lösung von 30 g (0.440 Mol) Kaliumhydroxid in 40 ml Wasser versetzt und bei Raumtemperatur über Nacht gerührt. Anschließend wird in Wasser eingegossen, die Lösung mit tert.Butylmethylether extrahiert und die wässrige Phase mit konz Salzsäure angesäuert Das sich abscheidende Produkt wird in Ethylacetat aufgenommen und die organische Phase mit Wasser gewaschen. Nach dem Trocknen über Natriumsulfat wird das Lösungsmittel i. Vak. entfernt. Der Rückstand wird zweimal aus Acetonitril unter Zusatz von Methanol (5 Vol%), danach zweimal aus Acetonitril/Trichlormethan (2:1) umkristallisiert Farblose Kristalle von Schmp. 152-155 °C; Ausbeute 13.7 g (37%).
$C_{21}H_{24}O_6$ (372.4)
Massenspektrum: m/e = 328 (15 %), ($M^+$ - $CO_2$) (kein $M^+$)
IR-Spektrum (KBr): $\nu$ (OH) 3600-2300 $cm^{-1}$
$\nu$ (C=O) 1690 $cm^{-1}$
$^1$H-NMR-Spektrum ($CDCl_3/CD_3OD$):
1.57 s (6) $(C\underline{H}_3)_2C$
1.73 bis 2.10 m (2) $ArCH_2C\underline{H}_2$

2.50 bis 3.08 m (2) $ArC\underline{H_2}CH_2$
3.84 bis 4.06 m (3) $C\underline{H_2}C\underline{H}O$
4.85 breites s (3) 3 $O\underline{H}$
6.80 bis 8.10 m (8) Aromat

Für die therapeutische Anwendung als hypolipämische Arzneimittel werden die neuen Verbindungen der allgemeinen Formel (1) und ihre Salze bevorzugt oral verabreicht. Gewöhnlich beträgt die orale Tagesdosis bei Erwachsenen 0.1 bis 5 g, vorzugsweise 0.3 bis 2 g.

Die Wirkstoffe können zur oralen Verabreichung in üblicherweise galenisch verarbeitet werden.Als pharmazeutische Trägerstoffe eignen sich gängige Hilfsstoffe wie Lactose,Saccharose,Mannit, Kartoffel- oder Maisstärke,Cellulosederivate oder Gelatine,gegebenenfalls unter Zusatz von Gleitmitteln, wie z.B. Magnesium- oder Calciumstearat, sowie Polyethylenglykole.

Beispiel

250 g der Verbindung von Beispiel 1 werden mit 250 g Polyethylenglykol vermischt und nach dem Schererverfahren in eintausend Weichgelatinekapseln abgefüllt, die je 250 mg Wirkstoff enthalten.

Bevorzugte Verabreichungsformen sind Steckkapseln aus Eartgelatine sowie geschlossene Weichkapseln. In Steckkapseln kann gegebenenfalls der reine Wirkstoff evtl. mit einem geringen Zusatz an Gleitmitteln enthalten sein. Bei entsprechenden physikochemischen Eigenschaften des Wirkstoffes wird eine Verarbeitung zu Granulaten bevorzugt, wobei als Hilfsstoffe Kartoffel- oder Maisstärke, mikrokristalline Cellulose,Cellulosederivae,Gelatine oder auch hochdisperse Kieselsäuren mitverwendet werden.

Bei Konfektionierung in Weichgelatinekapseln wird der reine Wirkstoff in geeigneten Flüssigkeiten gelöst oder suspendiert, so z.B. in flüssigen Polyethylenglykolen oder Pflanzenölen.

Pharmakodynamische Effekte der beanspruchten Verbindungen

Die Überlegenheit der beanspruchten Verbindungen gegenüber dem bereits seit langem in die Therapie eingeführten CLOFIBRAT läßt sich anhand der lipidsenkenden Wirkung eindeutig belegen.

1) Bestimmung der hypolipämischen Wirkung an Ratten

Die lipidsenkendeWirkung der Testsubstanzen wurde an Gruppen von jeweils 10 normolipämischen,männlichen, 200 bis 220 g schweren Wistar Ratten geprüft.

Die Durchführung der Untersuchungen erfolgte nach einem 3-wöchigen Umtrainieren der Fressgewohnheiten der Tiere. Die kontrollierte Fütterung wurde täglich von $8^{oo}$ Uhr bis $10^{oo}$ Uhr durchgeführt. Die Applikation der Testsubstanzen erfolgte um $11^{oo}$ Uhr.

Die Testverbindungen wurden in einer wäßrigen Lösung von 0.25% Agar und 0.84% NaCl aufgenommen und oral verabreicht. Nach dreimaliger Applikation über einen Zeitraum von drei Tagen wurde den Tieren Blut entnommen.

Die Bestimmung von Cholesterin und Triglyceriden im Serum wurde mit Hilfe des Zentrifugalanalysers "Cobas-Bio" von Hoffman-La Roche vorgenommen.

Methoden:

a) Cholesterinbestimmung
CHOD-PAP-Methode, enzymatischer Farbtest nach J.Siedel et al [J.Clin.Chem.Clin.Biochem. 19,838-(1981)]
b) Triglyceridbestimmung
Enzymatische Spaltung der Triglyceride mit Hilfe spezieller Lipasen mit nachfolgender Bestimmung des freigewordenen Glycerins nach T.O. Tiffany et al [Clin.Chem. 20, 476 (1974)]

Tabelle 2

| Prozentuelle Änderung der Gesamtcholesterin (TC) - und Triglycerid(TG)-spiegel im Rattenserum nach oraler Applikation der Testsubstanzen | | | |
|---|---|---|---|
| Verbindungsnummer | Dosis (mg/kg) | % Änderung | |
| | | TC | TG |
| | | $\overline{X}$ ± SD | $\overline{X}$ ± SD |
| Vergleichssubstanz CLOFIBRAT | 100 | - 14.5 ± 12.0 | - 29..2 ± 19.4 |
| 1 | 10 | + 3.8 ± 16.4 | - 26.2 ± 15.1 |
| 1 | 30 | - 17.1 ± 13.9 | - 28.5 ± 9.0 |
| 1 | 100 | - 44.1 ± 10.8 | - 34.7 ± 8.6 |
| 2 | 100 | - 39.7 ± 12.4 | - 1.6 ± 21.4 |
| 3 | 100 | - 17.5 ± 6.2 | - 19.4 ± 9.7 |

2) Bestimmung der Hemmung der Cholesterinbiosynthese

3 g frisch präparierte Rattenleber wurden in 6 ml eisgekühltem Puffer homogenisiert und bei 20.000 ×g 20 Minuten lang zentrifugiert. Der Überstand wurde abgetrennt und für die nachfolgenden Bestimmungen verwendet.

Zu einer Mischung von 110 µl Überstand und 300 µl Inkubationslösung wurden 10 µl der methanolischen Testsubstanzlösung pipettiert und bei 37° C 20 Minuten lang inkubiert. Anschließend wurden 10 µl einer $^{14}C$-Acetat Lösung hinzupipettiert und weitere 30 Minuten inkubiert. Danach wurden zu der Mischung 500 µl ethanolische KOH-Lösung zugefügt, 60 Minuten bei 75°C weiter inkubiert und anschließend die Reaktion im Eisbad gestoppt. Um eine Ausbeutekorrektur für die nachfolgende Extraktion des entstandenen $^{14}C$-Ch°lesterins vornehmen zu können, wurde $^{3}H$-Cholesterin (ca. $10^{-3}$ µCi) dazupipettiert .Darüberhinaus wurden 500 µl einer ethanolischen Lösung von unmarkiertem Cholesterin zugefügt. Das Cholesterin der Mischung wurde zweimal mit je 1 ml Petrolether extrahiert, der Extrakt zur Trockne gebracht und mit 0.6 ml einer Ethanol/Aceton-Mischung (1/1) aufgenommen. Das gelöste Cholesterin wurde dann mittels 1.4 ml einer Digitoninlösung ( = 7 mg) ausgefällt . Nach 15 h bei 0° C wurde der Niederschlag abzentrifugiert, mit 0.5 ml Methanol/Eisessig (33/5) gelöst und mit 2 ml Methanol verdünnt. Die Radioaktivität des biosynthetisch gebildeten Cholesterins wurde in 12 ml Scintillationsflüssigkeit (DIMILUME) mittels eins Scintillations-Zählers bestimmt.

Verwendete Lösungen :

Puffer : 6.8 g $KH_2PO_4$, 0.6 g $MgCl_2$ , 0.37 g EDTA, 1.83 g Nicotinamid, 51.35 g Saccharose und 0.44 g Mercaptoethanol wurden in destilliertem Wasser gelöst und auf 500 ml verdünnt [ $p_H$ = 7.2 ]
Inkubationslösung : Jeweils einzeln aufgelöst wurden :
8.6 mg NAD mit 1.3 ml Puffer
1o.2 mg NADP mit 1.3 ml Puffer
39.6 mg Glucose-6-phosphat mit 0.65 ml Puffer
31.6 mg Glucose-6-phosphat-Dehydrogenase (1000 U/0.9 ml) mit 0.285 ml Puffer
Diese Lösungen wurden erst unmittelbar vor der Verwendung vermischt. $^{14}C$-Acetat :
Lösung 1 : 250 µCi $^{14}C$-Natriumacetat in 1 ml Aqua dest.
Lösung 2 : 512.5 mg Natriumacetat in 10 ml Aqua dest.

Vor dem Versuch wurden 0.1 ml der Lösung 1 und 0.4 ml der Lösung 2 gemischt und mit 2 ml Aqua dest. versetzt.
Ethanolische KOH : 10 g KOH wurden in 100 ml Ethanol gelöst
Methode : R. E. Dugan et al [Archives Biochem.Biophys. 152,21(1972)]

Tabelle 3

| Prozentuelle Hemmung der Cholesterinbiosynthese im Rattenleberhomogenat durch die Testsubatanzen | | |
|---|---|---|
| Verbindungsnummer | Konzentration (Mol/l) | Hemmung x) % |
| 1 | $3 \times 10^{-6}$ | 0 |
| 1 | $1 \times 10^{-5}$ | 34 |
| 1 | $3 \times 10^{-5}$ | 57 |
| 1 | $1 \times 10^{-4}$ | 68 |
| 2 | $3 \times 10^{-6}$ | 16 |
| 2 | $1 \times 10^{-5}$ | 41 |
| 2 | $3 \times 10^{-5}$ | 65 |
| 2 | $1 \times 10^{-4}$ | 75 |
| 3 | $3 \times 10^{-6}$ | 14 |
| 3 | $1 \times 10^{-5}$ | 29 |
| 3 | $3 \times 10^{-5}$ | 56 |
| 3 | $1 \times 10^{-4}$ | 70 |

x) Prozentuelle Hemmung der Cholesterinbiosynthese gegenüber dem Leerwert.

## Patentansprüche

**1.** p-Oxibenzoesäurederivate der allgemeinen Formel (1)

(1) 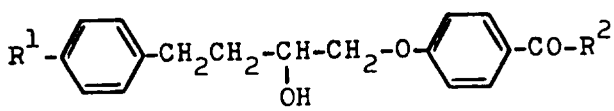

in der bedeuten:

$R^1$ =

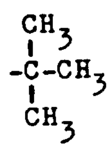 , 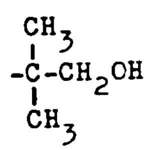 oder 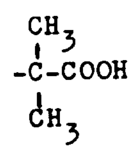

und $R^2$ = -OH ,

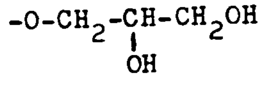 oder 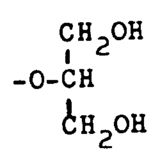 )

wobei im Falle von $R^1$ =

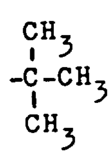

$R^2$ =

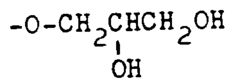 oder 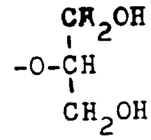

bedeutet und im Falle von $R^1$ =

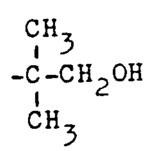 oder 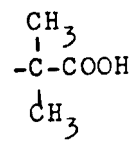

$R^2$ = -OH bedeutet, ihre physiologisch verträglichen Salze sowie ihre enantiomeren als auch diastereomeren Formen.

2. Verfahren zur Herstellung von p-Oxibenzoesäurederivaten nach Anspruch 1, dadurch gekennzeichnet, dar man in an sich bekannter Weise, Epoxide der allgemeinen Formel (2)

(2) 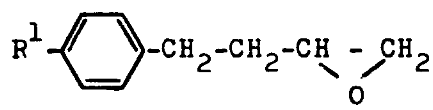

in der $R^1$ =

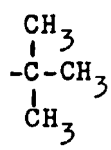 , 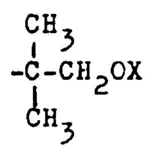 oder 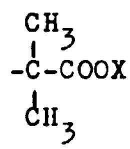

darstellt und X eine geeignete Schutzgruppe bedeutet mit den entsprechenden p-Oxibenzoesäureestern umsetzt und die gegebenenfalls vorhandenen Schutzgruppen abspaltet.

3. Verwendung von p-Oxibenzoesärederivaten nach Anspruch 1,
zur Herstellung eines Arzneimittels mit hypolipämischer Wirkung .

## Claims

1. p-Oxybenzoic acid derivatives of the general formula (1)

(1) 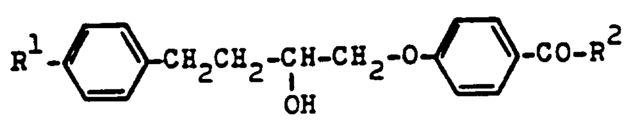

in which represent:
$R^1$ =

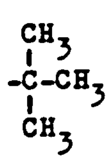 , 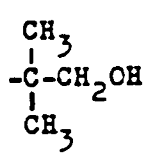 or 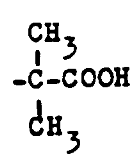

and

$R^2$ = -OH ,

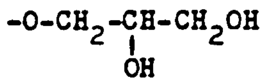 or 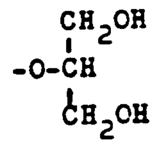 )

wherein in case of $R^1$ =

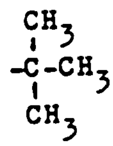

$R^2$ is represented by

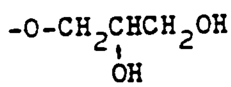 or 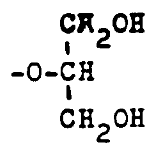

and in case of $R^1$ =

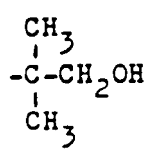 or 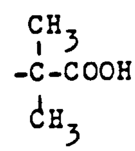

$R^2$ is represented by -OH ,

the physiologically acceptable salts and the enantiomeric and diastereomeric forms thereof.

**2.** A process for the preparation of the p-oxybenzoic acid derivatives according to claim 1, characterized in that epoxides of the general formula (2)

(2) 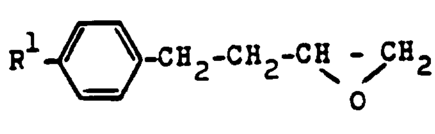

wherein $R_1$ is represented by

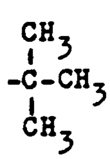 , 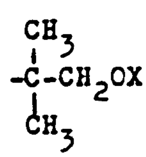 or 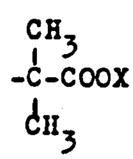

and X is a suitable protecting group,
are reacted with the corresponding p-oxybenzoic acid esters in a known manner and the eventually present protecting groups are cleaved off.

**3.** Use of the p-oxybenzoic acid derivatives according to claim 1 for the preparation of a medicament having hypolipaemic activity.

**Revendications**

**1.** Dérivés d'acides p-oxybenzoïques de formule générale (1)

(1) 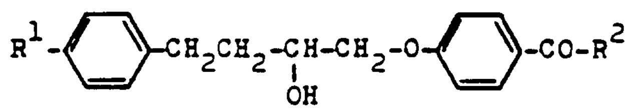

dans laquelle :
$R^1$ représente un groupe

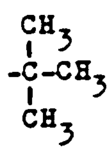 , 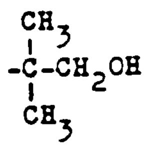 ou 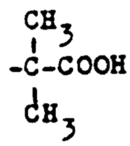

et $R^2$ représente un groupe -OH ,

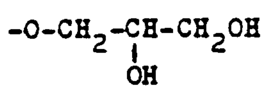 ou 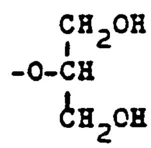 ,

et, si $R^1$ représente

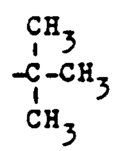

$R^2$ représente un groupe

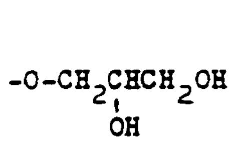 ou 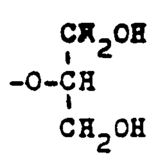

et, si $R^1$ représente

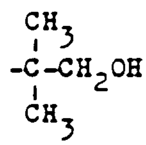 ou 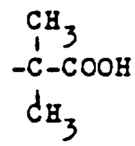

$R^2$ représente -OH. ,
leurs sels physiologiquement compatibles ainsi que leurs énantiomères et leurs formes diastéréomères.

**2.** Procédé de préparation de dérivés d'acides p-oxybenzoïques selon la revendication 1, caractérisé en ce qu'on fait réagir de façon connue en soi, des époxydes de formule générale (2)

(2) 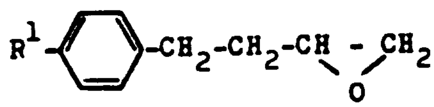

dans laquelle
$R^1$ représente un groupe

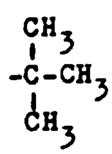 , 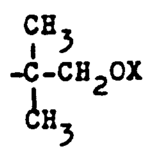 ou 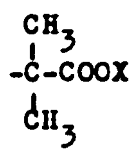 ,

et X représente un groupe protecteur convenable, avec les esters d'acides p-oxybenzoïques correspondants et l'on scinde les groupes protecteurs éventuellement présents.

**3.** Utilisation de dérivés d'acides p-oxybenzoïques selon la revendication 1 pour préparer des médicaments à action hypolipémiante.